# EUROPEAN PATENT APPLICATION

(11) **EP 4 527 874 A1**
(43) Date of publication of application: **26.03.2025**
(21) Application number: 23807410.8
(22) Date of filing: 27.04.2023
(51) Int. Cl.: C08G 77/26, A61K 8/19, A61Q 1/00, A61Q 1/10, A61Q 1/12, A61Q 17/04, A61Q 19/00, C08G 77/54

(54) **ORGANOPOLYSILOXANE, POWDER TREATMENT AGENT, AND TREATED POWDER AND COSMETIC TREATED WITH POWDER TREATMENT AGENT**

(30) Priority: 18.05.2022 JP 2022081745
(71) Applicant: SHIN-ETSU CHEMICAL CO., LTD., Tokyo 1000005 (JP)
(72) Inventor: MORIYA Hiroyuki, Annaka-shi, Gunma 379-0224 (JP)
(74) Representative: Angerhausen, Christoph
(86) International application number: PCT/JP2023/016567
(87) International publication number: WO 2023/223800

(57) **Abstract**

Provided is an organopolysiloxane suitable as a powder treatment agent exhibiting enhanced long-term storage stability and high reactivity with a powder surface. The organopolysiloxane is represented by average structural formula (1). 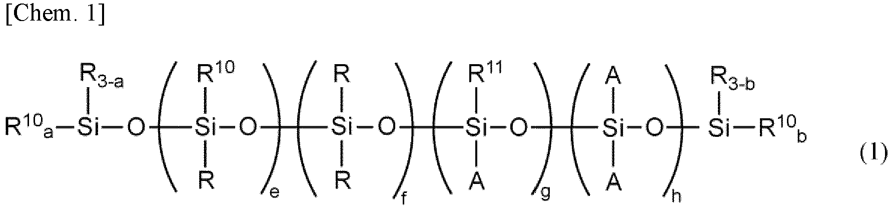 (In formula (1), R represents an alkyl group or the like, R¹⁰ is a polyoxyalkylene group which has a hydrolyzable silyl group at the terminal and of which there are one or more per molecule, R¹¹ is a group selected from R and R¹⁰, A is an organopolysiloxane segment having a specific structure, a and b are independently a number of 0 to 3, e is a number of 0 to 50, f is a number of 0 to 2,000, g is a number of 0 to 10, and h is 0 or 1. The bonding arrangement of siloxane units each identified by e, f, g, and h in brackets may be blocked or random.)

## Description

### Technical Field

The present invention relates to an organopolysiloxane having a hydrolyzable silyl group via a specific linking group, a powder treatment agent, and a treated powder and a cosmetic product using the same.

### Background Art

Treatment agents having a hydrolyzable silyl or hydrosilyl group, serving as a reaction site with powder surfaces, have been discussed as powder treatment agents for powder to be formulated in cosmetic products. An organopolysiloxane group and a polyoxyalkylene group for use respectively as a hydrophobicity-imparting group and a hydrophilicity-imparting group in the powder surface treatment using this treating agent have been investigated.

For example, Patent document 1 discloses a hydrophilized powder that is treated with a polyether-modified silicone having a hydrolyzable silyl group. Patent document 1 discusses the dispersibility of this treated powder into an aqueous material but no report can be found about the successful formation of a Pickering emulsion. Further, the polyether-modified silicone serving as a treatment agent has a hydrolyzable silyl group that is directly bonded to a silicon atom of the siloxane bond in the main chain, which causes significant steric hindrance and low reactivity between the treatment agent and powder, and therefore results in an inferior long-term storage stability. Furthermore, this hydrophilized powder has an issue in deterioration in usability such as stability over time and stickiness when the hydrophilized powder is formulated in a cosmetic product.

In addition, Patent document 2 reports that a treated powder using an organopolysiloxane having a hydrosilyl group and a polyoxyalkylene or polyglyceryl group exhibits good dispersibility into water and has emulsifiability of oily components. However, this organopolysiloxane has poor stability over time and undergoes gelation due to crosslinking reactions between hydrosilyl groups. Moreover, these hydrosilyl groups exhibit low reactivity to the powder surface, and therefore the powder surface is less likely to be treated in a sufficient manner, resulting in unsatisfactory dispersibility or emulsion stability. Furthermore, unreacted hydrosilyl groups may react with moisture to produce hydrogen gas, potentially having a detrimental effect on the stability or swelling of cosmetic containers.

### Prior Art Documents

### Patent documents

Patent document 1: JP-A-2004-155978
Patent document 2: JP-A-2010-30954

### Summary of Invention

### Technical Problem

It is therefore an object of the present invention to provide an organopolysiloxane that is suitable as a powder treatment agent exhibiting enhanced long-term storage stability and high reactivity with a powder surface. It is also an object of the present invention to provide a treated powder that can stabilize a water-in-oil Pickering emulsion. It is further an object of the present invention to provide a cosmetic product formulated with said Pickering emulsion.

### Solution to Problem

The inventor of the present invention diligently conducted a series of studies to solve the above problems, and found out that an organopolysiloxane represented by an average structural formula (1) as defined below can solve the above objects, thus completing the invention. That is, the present invention provides an organopolysiloxane as set forth below.

<1> An organopolysiloxane represented by the following average structural formula (1): 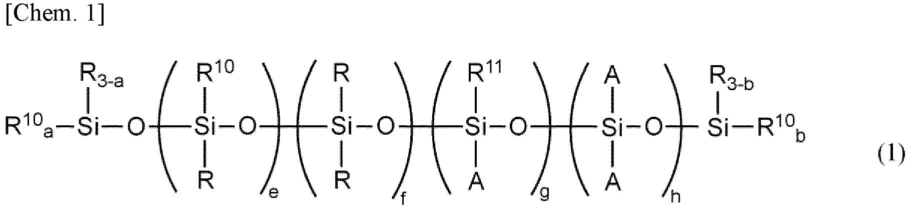
   wherein, in the formula (1), each R independently represents a group selected from a hydrogen atom, a hydroxy group, an alkyl group having 1 to 30 carbon atoms, a fluoroalkyl group having 1 to 12 carbon atoms, an aryl group having 6 to 12 carbon atoms, and an aralkyl group having 7 to 12 carbon atoms, and each R¹⁰ independently represents a group defined by the following average structural formula (2): 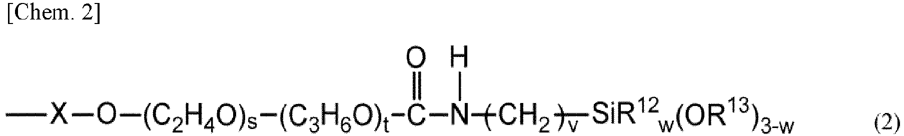 wherein, in the formula (2), X is a divalent hydrocarbon group having 1 to 10 carbon atoms, s is a number of 1 to 100, t is a number of 0 to 50, the bonding arrangement of oxyalkylene units each identified by s and t in brackets may be blocked or random, v is a number of 1 to 10, each R¹² independently represents a group selected from an alkyl group having 1 to 6 carbon atoms and an aryl group having 6 to 12 carbon atoms, each R¹³ independently represents a hydrogen atom or a monovalent hydrocarbon group having 1 to 6 carbon atoms, and w is a number of 0 to 2;
   wherein R¹¹ is a group selected from R¹⁰ or R, and each A independently represents an organopolysiloxane segment defined by the following average structural formula (3) or (4): 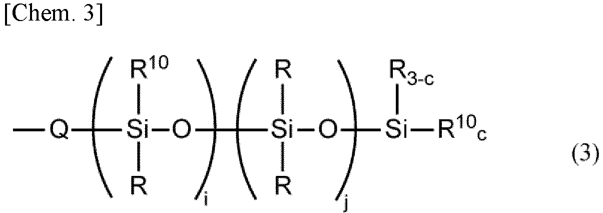 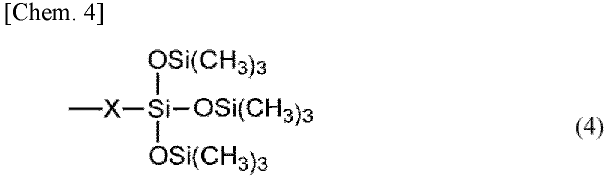 wherein, in the formula (3), R and R¹⁰ are as defined above, c is a number of 0 to 3, i is a number of 0 to 10, j is a number of 0 to 100, and Q is an oxygen atom or a divalent hydrocarbon group having 1 to 3 carbon atoms, and in the formula (4), X is as defined above, and the bonding arrangement of siloxane units each identified by i and j in brackets may be blocked or random, and
   wherein, in the formula (1), each of a and b independently represents a number of 0 to 3, e is a number of 0 to 50, f is a number of 0 to 2,000, g is a number of 0 to 10, h is 0 or 1, the bonding arrangement of siloxane units each identified by e, f, g and h in brackets may be blocked or random, and one or more R¹⁰s are present in the formula (1).
<2> A powder treatment agent comprising the organopolysiloxane according to <1>, wherein the agent contains water in an amount of 2,000 ppm or less.
<3> A powder surface treated with the powder treatment agent according to <2>.
<4> The treated powder according to <3>, wherein the powder is an organic or inorganic powder.
<5> A cosmetic product containing the treated powder according to <4>.
<6> The cosmetic product according to <5>, further comprising water and an oil agent.

### Advantageous Effects of Invention

The organopolysiloxane of the present invention, containing a group in which a polyoxyalkylene group and a hydrolyzable silyl group are bonded via urethane binding, exhibits enhanced long-term storage stability and high reactivity with a powder surface. Further, a treated powder obtained via surface treatment using the organopolysiloxane of the present invention may be arranged between an interface between water and oil agent to form a Pickering emulsion, which particularly enables the formation of a water-in-oil Pickering emulsion which has been difficult to stabilize. Accordingly, a cosmetic product containing the treated powder exhibits no stickiness or irritation peculiar to a surfactant agent, forms a uniform cosmetic layer, exhibits favorable spreading, glideability, and adhesiveness when it is applied, and exhibits excellent usability and stability over time.

### Description of Embodiments

The present invention will be described in detail hereunder but the invention shall not be limited to the following.

### [Organopolysiloxane]

The organopolysiloxane of the present invention is an organopolysiloxane represented by an average structural formula (1) as defined below.

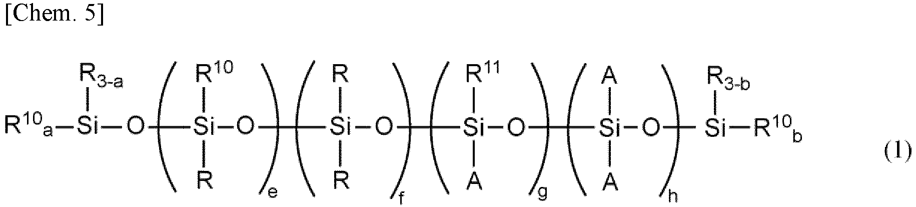

In the formula (1), each R independently represents a group selected from a hydrogen atom, a hydroxy group, an alkyl group having 1 to 30 carbon atoms, a fluoroalkyl group having 1 to 12 carbon atoms, an aryl group having 6 to 12 carbon atoms, and an aralkyl group having 7 to 12 carbon atoms. R is preferably a group selected from an alkyl group having 1 to 16 carbon atoms, a fluoroalkyl group having 1 to 10 carbon atoms, and a phenyl group, and most preferably is a methyl group.

In the formula (1), each R¹⁰ is a group represented by an average structural formula (2) as defined below, and the present invention is characterized by having one or more, preferably 1 to 50, more preferably 2 to 20 R¹⁰s per one molecule of the organopolysiloxane represented by the average structural formula (1). As shown by an average structural formula (2) as defined below, R¹⁰ is a group having a hydrolyzable silyl group at its end, where the hydrolyzable silyl group is bonded to a polyoxyalkylene group via urethane bonding.

The R¹⁰ may be positioned at an end of the siloxane molecular chain, as shown in the average structural formula (1), or in midway through the chain (at a side chain of the molecular chain) or be positioned at either one or both of them.

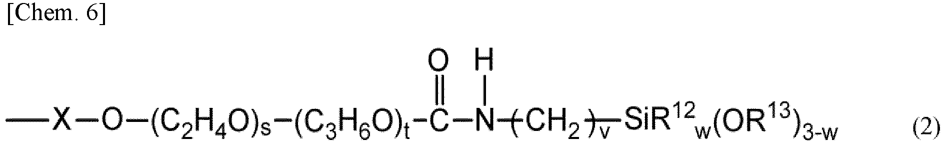

In the formula (2), X is a divalent hydrocarbon group having 1 to 10, preferably 2 to 8, carbon atoms, and most preferably is a trimethylene group or a 2-methyl trimethylene group.

In the formula (2), s represents a number of 1 to 100, preferably 4 to 20. t represents a number of 0 to 50, preferably 0 to 20. s + t is preferably 4 to 50, more preferably 8 to 20. A value of s + t kept within this range allows a powder to have sufficient hydrophilicity when the powder is treated, and is therefore preferable. Further, the bonding arrangement of oxyalkylene units each identified by s and t may be blocked or random.

In the formula (2), v is a number of 1 to 10, preferably 1 to 6, and it is more preferred that v = 1, v = 2, v =3, or v = 6, and it is even more preferred that v = 3.

In the formula (2), R¹² is a group selected from an alkyl group having 1 to 6 carbon atoms and an aryl group having 6 to 12 carbon atoms, and is preferably a methyl group.

In the formula (2), R¹³ is a hydrogen atom or a monovalent hydrocarbon group having 1 to 6 carbon atoms, and is preferably a hydrogen atom, a methyl group, an ethyl group, or a propyl group.

In the formula (2), w is a number of 0 to 2, and is preferably 0 or 1, and more preferably 0.

In the formula (1), R¹¹ is a group selected from R¹⁰ and R, and A is an organopolysiloxane segment represented by an average structural formula (3) or (4) as defined below, and among them, it is preferred that R¹¹ be a group represented by the following formula (3).

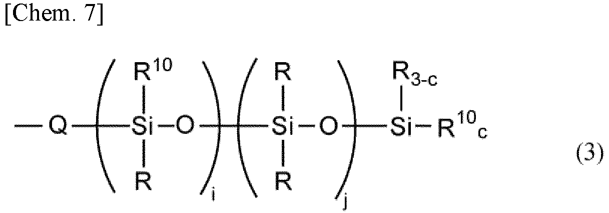

In the formula (3), R and R¹⁰ are as defined above, Q is an oxygen atom or a divalent hydrocarbon group having 1 to 3 carbon atoms and is preferably an oxygen atom or an ethylene group. In the formula (3), c is a number of 0 to 3. i is a number of 0 to 10, preferably of 0 to 5. j is a number of 0 to 100, preferably of 0 to 50. The values of i and j respectively kept within this range allow favorable reactivity with powder, and therefore is preferable.

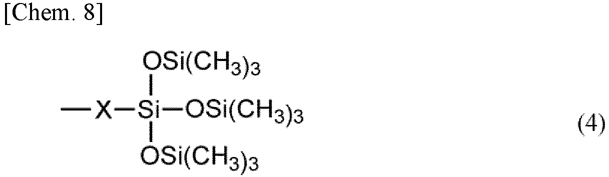

In the formula (4), X is as defined above.

In the formula (1), each of a and b independently represents a number of 0 to 3. e is a number of 0 to 50, preferably of 1 to 30, and more preferably of 2 to 20. f is a number of 0 to 2,000, preferably of 1 to 1,000, and more preferably of 5 to 100. Values of e and f respectively kept within this range allow favorable reactivity with powder, and therefore is preferable. g is a number of 0 to 10, preferably of 0 to 8, and more preferably of 0 to 5. h is a number of 0 or 1.

Examples of the method of manufacturing an organopolysiloxane of the present invention include a method of allowing a polyoxyalkylene-modified organopolysiloxane, having a hydroxy group at its end, and a isocyanate, having a hydrolyzable silyl group, to be reacted with each other.

Examples of the polyoxyalkylene-modified organopolysiloxane having a hydroxy group at its end include an organopolysiloxane represented by an average structural formula (1') as defined below.

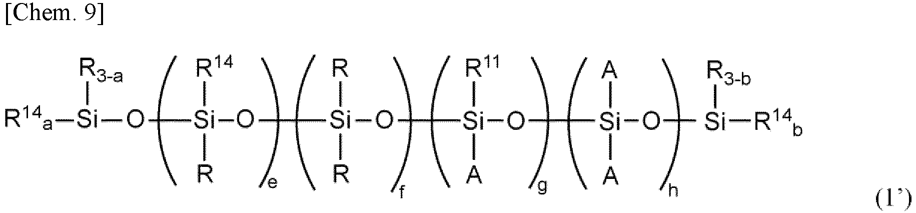

In the formula (1'), R, R¹¹, A, a, b, e, f, g, and h are same as those defined in the formula (1), R¹⁴ is a group represented by an average structural formula (5) as defined below, and, the bonding arrangement of siloxane units each identified by e, f, g and, h in brackets may be blocked or random. Here, one or more R¹⁴s are present in the formula (1').

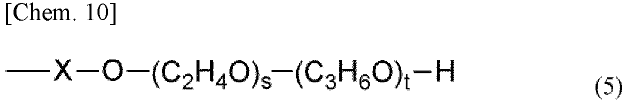

In the formula (5), X, s, and t are all the same as those defined in the formula (2), and the bonding arrangement of oxyalkylene units each identified by s and t in brackets may be blocked or random.

Examples of the isocyanate having a hydrolyzable silyl group include an isocyanate represented by a formula (6) as defined below.

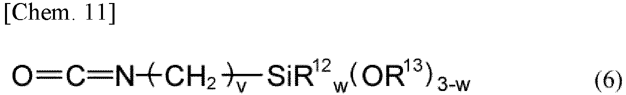

In the formula (6), R¹², R¹³, v, and w are all the same as those defined above.

Further, at the time of reaction, there may be used a solvent, as necessary, which is not particularly limited as long as the solvent is an aprotic organic solvent. Examples of the solvent include an aromatic hydrocarbon such as toluene and xylene; an aliphatic or alicyclic hydrocarbon such as n-pentane, n-hexane and cyclohexane; and a halogenated hydrocarbon such as dichloromethane, chloroform and carbon tetrachloride. The reaction conditions are not particularly limited, but a reaction temperature of 50 to 120°C and a reaction time of 1 to 10 hours are preferred.

Further, the reaction may be performed in the presence of a urethane formation catalyst. Examples of the urethane formation catalyst include known catalysts used for forming urethane bonds such as, amines including triethylamine, triethylenediamine, and N-methylmorpholine; and organometallic compounds including di-n-butyltin dilaurate and stannous oleate.

Furthermore, it is preferable to distill away unreacted materials, catalysts, and water content under reduced pressure at 80 to 120°C after performing such urethane formation reaction. The organopolysiloxane having water content reduced to 2,000 ppm or less, preferably 1,800 ppm or less enhances long-term storage stability, which is therefore preferable when used as a powder treatment agent. The water content in the organopolysiloxane as used herein is a value measured using a Karl Fischer moisture meter.

### [Powder Treatment Agent/Treated Powder]

The organopolysiloxane of the present invention may be suitably used as a powder treatment agent. When the organopolysiloxane is used as a powder treatment agent, it is preferred that the organopolysiloxane have a water content of 2,000 ppm or less, more preferably 1,800 ppm or less.

Examples of powders to be treated with the powder treatment agent include an inorganic powder, an organic powder, a surfactant metal salt powder (metal soap), a colored pigment, a pearl pigment, and a metal powder pigment.

Examples of the inorganic powder include titanium oxide, zirconium oxide, zinc oxide, cerium oxide, magnesium oxide, barium sulfate, calcium sulfate, magnesium sulfate, calcium carbonate, magnesium carbonate, talc, mica, kaolin, sericite, muscovite, synthetic mica, phlogopite, red mica, biotite, lithia mica, silicic acid, anhydrous silicic acid, aluminum silicate, magnesium silicate, aluminum magnesium silicate, calcium silicate, barium silicate, strontium silicate, metal tungstate, hydroxyapatite, vermiculite, Higilite, bentonite, montmorillonite, hectorite, zeolite, ceramic powder, dibasic calcium phosphate (calcium monohydrogen phosphate), alumina, aluminum hydroxide, boron nitride, and silica. Preferable examples thereof include titanium oxide, zinc oxide talc, mica, kaolin, sericite, muscovite, synthetic mica, phlogopite, red mica, biotite, lithia mica, silicic acid, anhydrous silicic acid, bentonite, montmorillonite, hectorite, zeolite, ceramic powder, alumina, aluminum hydroxide, boron nitride, and silica.

Examples of the organic powder include a polyamide powder, a polyester powder, a polyethylene powder, a polypropylene powder, a polystyrene powder, a polyurethane powder, a benzoguanamine powder, a polymethyl benzoguanamine powder, a tetrafluoroethylene powder, a polymethylmethacrylate powder, a cellulose powder, a silk powder, a nylon powder, a 12 nylon powder, a 6 nylon powder, a silicone powder, a silicone composite powder, a styrene-acrylic acid copolymer powder, a divinylbenzene-styrene copolymer powder, a vinyl resin powder, a urea resin powder, a phenolic resin powder, a fluorine carbon resin powder, a silicone resin powder, an acrylic resin powder, a melamine resin powder, an epoxy resin powder, a polycarbonate resin powder, a microcrystalline fiber powder, a starch powder, and a lauroyl lysine powder. Preferable examples thereof include a polyethylene powder, a polypropylene powder, a polyurethane powder, a polymethylmethacrylate powder, a cellulose powder, a silk powder, a nylon powder, a silicone powder, a silicone composite powder, an acrylic resin powder, and a starch powder.

Examples of the surfactant metal salt powder (metallic soap) include zinc stearate, aluminum stearate, calcium stearate, magnesium stearate, zinc myristate, magnesium myristate, zinc cetyl phosphate, calcium cetyl phosphate, and sodium zinc cetyl phosphate. Examples of the colored pigment include, for example, an inorganic red pigment such as iron oxide, iron hydroxide, and iron titanate; an inorganic brown pigment such as γ-iron oxide; an inorganic yellow pigment such as yellow iron oxide and loess; an inorganic black pigment such as black iron oxide and carbon black; an inorganic purple pigment such as manganese violet and cobalt violet; an inorganic green pigment such as chromium hydroxide, chromium oxide, cobalt oxide, and cobalt titanate; an inorganic blue pigment such as Prussian blue and ultramarine; a tar-based pigment that has been turned into a lake pigment; a natural pigment that has been turned into a lake pigment; and a composite powder prepared by compounding these powders.

Examples of the pearl pigment include, for example, titanium oxide-coated mica, bismuth oxychloride, titanium oxide-coated bismuth oxychloride, titanium oxide-coated talc, fish scale foil, and titanium oxide-coated colored mica. An example of the metal powder pigment includes, for example, a powder selected from an aluminum powder, a copper powder, and a stainless-steel powder.

Any one of them can be used regardless of their shapes, particle diameters, or particle structures so as long as it is the one that is commonly used, but it is preferably spherical and has a particle size in a range of 5 nm to 1 µm.

The method of manufacturing the treated powder of the present invention involves treating the powder with a powder treatment agent containing the organopolysiloxane, and a dry processing or a wet processing, for example, may be selected therefor. A bead mill, a hammer mill, a Nauta mixer, or other machine can be used therefor.

It is preferred that the surface treatment be performed under heat to make the hydrolyzable alkoxy silyl groups of the powder treatment agent well react with a powder surface. The temperature is set at 30 to 150°C, particularly preferably at 30 to 110°C. The treatment time is preferably 5 minutes to 10 hours.

The powder treatment agent may be used in an amount of 0.1 to 30 parts by mass, preferably 0.5 to 10 parts by mass of the organopolysiloxane represented by the formula (1) in the powder treatment agent per 100 parts by mass of the powder. Furthermore, an optional component such as an organic solvent may be added as necessary when it is treated with the surface treatment agent.

Furthermore, in order to obtain a desired treated powder, a further treatment step, such as stirring, grinding, and drying, may be added as necessary on top of the above-mentioned treatment steps.

### [Cosmetic Product]

The present invention also provides a cosmetic product (hereinafter also referred to as "cosmetic product of the present invention") containing a powder (treated powder) that is surface treated with the powder treatment agent. The cosmetic product may contain not only the treated powder but also a medium that is allowable for cosmetics.

The treated powder of the present invention may be contained in an amount of preferably 0.1 to 90% by mass, more preferably 0.2 to 50% by mass, and even more preferably 0.5 to 20% by mass, per the whole cosmetic product.

It is preferred that the cosmetic product of the present invention further contain water and an oil agent on top of the above-mentioned treated powder. This allows the cosmetic product of the present invention to have favorable emulsion stability and favorable usability.

The water content thereof may be in an amount of preferably 1 to 98% by mass, more preferably 2 to 90% by mass, and even more preferably 3 to 80% by mass, per the whole cosmetic product. A water content kept within the above range results in a cosmetic product that is favorable in respect of stability and usability. The content of the oil agent may be in an amount of preferably 1 to 98% by mass, more preferably 2 to 50% by mass, and even more preferably 3 to 40% by mass, per the whole cosmetic product. An oil agent content kept within the above range results in a cosmetic product that is favorable in respect of stability and usability.

In addition, the cosmetic product of the present invention may further include a hydrophilic substance selected from propylene glycol, trimethylene glycol, dipropylene glycol, 1,3-butylene glycol, methylpropanediol, pentylene glycol, glycerin, diglycerin, ethylhexylglycerin, triglycerin, polyglycerin, and ethanol. It is preferred that each of these hydrophilic substances be in an amount of 1 to 80% by mass, more preferably 1 to 40% by mass, and even more preferably 3 to 30% by mass, per the whole cosmetic product. The hydrophilic substance content kept within the above range results in a cosmetic product that is favorable in respect of stability and usability.

Examples of the oil agent contained in the cosmetic product of the present invention include a volatile oil agent, such as a volatile silicone oil and a volatile organic oil, and an oil agent acceptable for use in cosmetic products other than the volatile oil agents.

Examples of the volatile silicone oil include, for example, dimethylpolysiloxanes (dimer, trimer, tetramer, pentamer), caprylyl methicone, octamethylcyclotetrasiloxane, decamethylcyclopentasiloxane, dodecamethylcyclohexasiloxane, tetramethyltetrahydrogencyclotetrasiloxane, tristrimethylsiloxymethylsilane, and tetrakistrimethylsiloxysilane.

Examples of the volatile organic oil include a hydrocarbon oil and an ester oil, and specific examples thereof include, for example, α-olefin oligomer, light isoparaffin, isododecane, light liquid isoparaffin, and butyl acetate.

Preferable examples of the volatile oil include dimethylpolysiloxanes (tetramer, pentamer), caprylyl methicone, tristrimethylsiloxymethylsilane, tetrakistrimethylsiloxysilane, light isoparaffin, isododecane, light liquid isoparaffin, and butyl acetate.

The content of these volatile oil agents may be in an amount of preferably 1 to 98% by mass, more preferably of 2 to 50% by mass, and even more preferably of 3 to 30% by mass, per the whole cosmetic product.

Examples of such oil agent other than the above-mentioned volatile oil agents include one or more non-volatile silicone oils, non-volatile hydrocarbon oils, non-volatile polar oils, such as higher fatty acids, higher alcohols, ester oils, glyceride oils and natural animal/vegetable oils, non-volatile semisynthetic oils, and non-volatile fluorinated oils.

Examples of the nonvolatile silicone oil include, for example, non-volatile ones such as linear or branched organopolysiloxanes having low viscosity to high viscosity, including dimethylpolysiloxane, phenyltrimethicone, methylphenylpolysiloxane, dimethylsiloxy phenyl trimethicone, diphenylsiloxy phenyl trimethicone (for example, KF-56A manufactured by Shin-Etsu Chemical Co., Ltd.), methylhexylpolysiloxane, methylhydrogenpolysiloxane, and dimethylsiloxane/methylphenylsiloxane copolymer; tetramethyltetraphenylcyclotetrasiloxane, amino-modified organopolysiloxane; silicone rubbers, such as highly polymerized gummy dimethylpolysiloxane, gummy amino-modified organopolysiloxane, and gummy dimethylsiloxane/methylphenylsiloxane copolymer; a solution of silicone gum or rubber in cyclic siloxane; trimethylsiloxy silicic acid, and a solution of trimethylsiloxy silicic acid in cyclic organopolysiloxane; higher alkoxy-modified organopolysiloxanes, such as stearoxy silicone; higher fatty acid-modified organopolysiloxane, alkyl-modified organopolysiloxane, long chain alkyl-modified organopolysiloxane, fluorine-modified organopolysiloxane, silicone resin, and silicone resin melts.

Examples of the nonvolatile hydrocarbon oil include, for example, nonvolatile ones, including ozocerite, squalane, synthetic squalane, vegetable squalane, squalene, ceresin, paraffin, paraffin wax, polyethylene wax, polyethylene/polypropylene wax, (ethylene/propylene/styrene) copolymer, (butylene/propylene/styrene) copolymer, liquid paraffin, liquid isoparaffin, pristane, polyisobutylene, hydrogenated polyisobutene, microcrystalline wax, and vaseline.

Examples of the nonvolatile higher fatty acid include, for example, lauric acid, myristic acid, palmitic acid, stearic acid, behenic acid, undecylenic acid, oleic acid, linoleic acid, linolenic acid, arachidonic acid, eicosapentaenoic acid (EPA), docosahexaenoic acid (DHA), isostearic acid, and 12-hydroxystearic acid.

Examples of the nonvolatile higher alcohol include, for example, lauryl alcohol, myristyl alcohol, palmityl alcohol, stearyl alcohol, behenyl alcohol, hexadecyl alcohol, oleyl alcohol, isostearyl alcohol, hexyl dodecanol, octyl dodecanol, cetostearyl alcohol, 2-decyl tetradecynol, cholesterol, phytosterol, POE cholesterol ether, monostearyl glycerin ether (batyl alcohol), and monooleyl glyceryl ether (selachyl alcohol).

Examples of the nonvolatile ester oil include, for example, diisobutyl adipate, 2-hexyldecyl adipate, di-2-heptylundecyl adipate, N-alkylglycol monoisostearate, isocetyl isostearate, trimethylolpropane triisostearate, ethylene glycol di-2-ethylhexanoate, cetyl 2-ethylhexanoate, trimethylolpropane tri-2-ethylhexanoate, pentaerythritol tetra-2-ethylhexanoate, cetyl octanoate, octyl dodecyl gum ester, oleyl oleate, octyldodecyl oleate, decyl oleate, neopentyl glycol dioctanoate, neopentyl glycol dicaprate, coco-(caprate/caprylate), triethyl citrate, 2-ethylhexyl succinate, isocetyl stearate, butyl stearate, diisopropyl sebacate, di-2-ethylhexyl sebacate, cetyl lactate, myristyl lactate, isononyl isononanoate, isotridecyl isononanoate, isopropyl palmitate, 2-ethylhexyl palmitate, 2-hexyldecyl palmitate, 2-heptylundecyl palmitate, cholesteryl 12-hydroxystearate, dipentaerythritol fatty acid ester, isopropyl myristate, octyldodecyl myristate, 2-hexyldecyl myristate, myristyl myristate, hexyldecyl dimethyloctanoate, ethyl laurate, hexyl laurate, N-lauroyl-L-glutamic acid-2-octyldodecyl ester, lauroyl sarcosine isopropyl ester, and diisostearyl malate.

Examples of the nonvolatile glyceride oil include, for example, acetoglyceryl, glyceryl triisooctanoate, glyceryl triisostearate, glyceryl triisopalmitate, glyceryl monostearate, glyceryl di-2-heptylundecanoate, glyceryl trimyristate, diglyceryl myristate isostearate, and glyceryl triethylhexanoate.

Examples of the nonvolatile natural animal/vegetable oils and semisynthetic oils include, for example, avocado oil, linseed oil, almond oil, Chinese wax, perilla oil, olive oil, cacao butter, kapok wax, kaya oil, carnauba wax, cod-liver oil, candelilla wax, purified candelilla wax, beef tallow, neats-foot oil, beef bone fat, cured beef tallow, apricot kernel oil, whale wax, hydrogenated oil, wheat germ oil, sesame oil, rice germ oil, rice bran oil, sugarcane wax, sasanqua oil, safflower oil, shea butter, Chinese tung oil, cinnamon oil, jojoba wax, squalane, squalene, shellac wax, turtle oil, soybean oil, tea seed oil, camellia oil, evening primrose oil, corn oil, pig fat, rapeseed oil, Japanese tung oil, bran wax, germ oil, horse fat, persic oil, palm oil, palm kernel oil, castor oil, cured castor oil, methyl ester of castor oil fatty acid, sunflower oil, grape seed oil, bayberry wax, jojoba oil, hydrogenated jojoba oil, macademia nut oil, bees wax, mink oil, meadowfoam seed oil, cotton seed oil, cotton wax, Japan wax, Japan wax kernel oil, montan wax, coconut oil, cured coconut oil, tri-coconut fatty acid glyceride, mutton tallow, peanut oil, lanolin, liquid lanolin, reduced lanolin, lanolin alcohol, hard lanolin, lanolin acetate, lanolin alcohol acetate, isopropyl lanolin fatty acid, and egg-yolk oil.

Examples of the nonvolatile fluorinated oil include, for example, perfluoro polyether, perfluoro decalin, and perfluoro octane.

The oil material that is allowable for the cosmetic product of the present invention, other than the volatile oil, may be blended in an amount of suitably 1 to 98% by mass, preferably 2 to 50% by mass, more preferably 3 to 30% by mass, per the whole cosmetic product, although the amount varies depending on the form of the cosmetic product.

The cosmetic product of the present invention can further contain one or more kinds of ultraviolet absorber and/or UV scattering agent. This makes the cosmetic product of the present invention not only have favorable usability, excellent handleability and persistency, but also capable of blocking ultraviolet ray.

Examples of the ultraviolet absorber include, for example, benzoic acid UV absorbers such as para-amino benzoic acid; anthranilic acid UV absorbers such as methyl anthranilate; salicylic acid UV absorbers such as methyl salicylate; cinnamic acid UV absorbers, such as ethylhexyl para-methoxycinnamate; benzophenone UV absorbers such as 2,4-dihydroxybenzophenone; urocanic acid UV absorbers such as ethyl urocanate; triazine UV absorbers such as bis-ethylhexyloxyphenol methoxyphenyl triazine; and dibenzoylmethane UV absorbers such as 4-t-butyl-4' -methoxy-dibenzoylmethane. It is also possible to use silicone derivatives having an UV-absorbing functional group described above.

Examples of the UV scattering agent include powder that absorb and scatter ultraviolet light, such as titanium oxide microparticles, iron-containing titanium oxide microparticles, zinc oxide microparticles, cerium oxide microparticles, and composites thereof. Among them, preferred are cinnamic acid UV absorbers, dibenzoylmethane UV absorbers, titanium oxide microparticles (for example, SPD-T5 manufactured by Shin-Etsu Chemical Co., Ltd.), and zinc oxide microparticles (for example, SPD-Z5 manufactured by Shin-Etsu Chemical Co., Ltd.; etc.).

The cosmetic product of the present invention may contain one or more kinds of surfactant. This makes the cosmetic product of the present invention a product that is more excellent in emulsification stability or usability which is tailored to its intended use. Examples of the surfactant includes anionic, cationic, nonionic, and amphoteric surfactants but the surfactant contained in the cosmetic product is not subjected to a particular limitation; i.e., any surfactant that is normally used for conventional cosmetics may be used.

Specific examples of the anionic surfactant include, for example, fatty acid soap, such as sodium stearate and triethanolamine palmitate; alkyl ether carboxylic acids and salts thereof; condensation salts of amino acid and fatty acid; alkane sulfonate salts; alkene sulfonate salts; sulfonate salts of fatty acid ester, sulfonate salts of fatty acid amide; sulfonate salts of formalin condensate; alkyl sulfate ester salts; sulfate ester salts of secondary higher alcohols; sulfate ester salts of alkyl and allyl ether; sulfate ester salts of fatty acid ester; sulfate ester salts of fatty acid alkylolamide; sulfate ester salts, such as Turkey red oil; alkyl phosphate salts; ether phosphate salts; alkyl allyl ether phosphate salts; amide phosphate salts; N-acyl lactate salt; N-acylsarcosine salts; N-acylamino acid activators; carboxy vinyl polymers; and polyacryl amide-based anionic surfactants.

Examples of the cationic surfactant include, for example, alkyl amine salts; amine salts of, for example, polyamines, amino alcohol fatty acid derivatives; alkyl quaternary ammonium salts (for example, behentrimonium chloride), aromatic quaternary ammonium salts, pyridinium salts, imidazolium salts.

Examples of the nonionic surfactant include, for example, sorbitan fatty acid esters, glycerin fatty acid esters, polyglycerin fatty acid esters, propylene glycol fatty acid esters, polyethylene glycol fatty acid esters, sucrose fatty acid esters, methyl glucoside fatty acid esters, alkyl polyglucosides, polyoxyethylene alkyl ethers, polyoxypropylene alkyl ethers, polyoxyethylene alkyl phenyl ethers, polyoxyethylene fatty acid esters, polyoxyethylene sorbitan fatty acid esters, polyoxyethylene sorbitol fatty acid esters, polyoxyethylene glycerin fatty acid esters, polyoxyethylene propylene glycol fatty acid esters, polyoxyethylene castor oil, polyoxyethylene hydrogenated castor oil, polyoxyethylene phytostanol ethers, polyoxyethylene phytosterol ethers, polyoxyethylene cholestanol ethers, and polyoxyethylene cholesterol ethers;
linear or branched polyoxyalkylene-modified organopolysiloxanes (for example, KF-6017, KF-6017P, KF-6028 manufactured by Shin-Etsu Chemical Co., Ltd.), linear or branched organopolysiloxanes co-modified with polyoxyalkylene and alkyl (for example, KF-6038, KF-6048 manufactured by Shin-Etsu Chemical Co., Ltd.), linear or branched polyglycerin-modified organopolysiloxanes (for example, KF-6100, KF-6104, KF-6106 manufactured by Shin-Etsu Chemical Co., Ltd.), linear or branched organopolysiloxanes co-modified with polyglycerin and alkyl (for example, KF-6105 manufactured by Shin-Etsu Chemical Co., Ltd.), alkanol amides, sugar ethers, and sugar amides.

Examples of the amphoteric surfactant include, for example, amphoteric surfactants of betaines, aminocarboxylic acid salts, imidazoline derivatives, and amide amine type.

The cosmetic product of the present invention may further contain a composition composed of liquid oil material and one or more kinds of crosslinked organopolysiloxane polymer having no hydrophilic group. The crosslinked organopolysiloxane polymer can be obtained by reaction of alkylhydrogenpolysiloxane and/or arylhydrogenpolysiloxane with a crosslinking agent that has a reactive unsaturated group(s) (reactive vinyl group(s)) at the terminal of the molecular chain.

Examples of the alkylhydrogenpolysiloxane include, for example, methylhydrogenpolysiloxane that is linear or having a partially branched unit and methylhydrogenpolysiloxane in which an alkyl chain with 6 to 20 carbon atoms is grafted. The methylhydrogen polysiloxane should have at least two, on average, hydrogen atoms that are bonded to silicon atoms per molecule. Examples of the crosslinking agent include a molecule having two or more reactive vinyl groups, per molecule, such as methylvinylpolysiloxane and α,ω-alkenyldiene.

Examples thereof include compositions described in JP-B-1925781, JP-B-1932769, a pamphlet of WO03/24413(A1), and JP-A-2009-185296. Such crosslinked organopolysiloxane polymer product may be swollen with, for example, a low-viscosity silicone with a viscosity of 0.65 to 100.0 mm²/s (at 25°C); a hydrocarbon oil, such as liquid paraffin, squalane, and isododecane; a glyceride oil, such as trioctanoin; and/or an ester oil in an amount of the own weight or more. Examples of commercial products of a composition composed of such crosslinked organopolysiloxane polymer product organopolysiloxane having no hydrophilic group and a liquid oil material include, but are not particularly limited to, for example, KSG-15, KSG-16, KSG-18, KSG-18A, KSG-1610, and USG-103, which are pastes mixed with silicone oil; USG-106, KSG-41, KSG-42, KSG-43, KSG-44, and KSG-810, which are pastes mixed with hydrocarbon oil or triglyceride oil (all of which are manufactured by Shin-Etsu Chemical Co., Ltd.).

The composition composed of a liquid oil material and the crosslinked organopolysiloxane having no hydrophilic group may be blended in an amount of preferably 0.1 to 50% by mass, further preferably 1 to 30% by mass, per the whole amount of the cosmetic.

The cosmetic product of the present invention may further contain a composition composed of a liquid oil material and one or more kinds of crosslinked organopolysiloxane polymer product having a hydrophilic group. The hydrophilic group is preferably a polyether group or a polyglycerin group. It may be obtained using a reaction of a crosslinking agent that has a crosslinked organo unsaturated group(s) (reactive vinyl group(s)) having a polyether group and/or a polyglycerin group.
Examples of which include an alkyllhydrogenpolysiloxane such as a methylhydrogenpolysiloxane in which a polyoxyethylene chain is grafted and a methylhydrogenpolysiloxane in which a polyglycerin chain is grafted. The molecule should have at least two, on average, hydrogen atoms that are bonded to silicon atoms per molecule. Examples of the crosslinking agent include a molecule having two or more vinyl reactive sites, per molecule, such as methylvinylpolysiloxane, α,ω-alkenyldienes, glycerol triallyl ether, polyoxyalkynylated glycerol triallyl ether, trimethylolpropane triallyl ether and polyoxyalkynylated trimethylolpropane triallyl ether, and the crosslinked product (crosslinked organopolysiloxane polymer product) obtained by reacting the above-mentioned alkylhydrogenpolysiloxane with a crosslinking agent is a compound having at least one hydrophilic group per molecule.

This crosslinked organopolysiloxane polymer product may be swollen with, for example, a low viscosity silicone having a viscosity of 0.65 to 100.0 mm²/s (at 25°C); a hydrocarbon oil, such as liquid paraffin, squalane, and isododecane; a glyceride oil such as trioctanoin; and/or an ester oil in an amount of the own weight or more.

Preferable examples of the composition include ones described in JP-B-2631772, JP-A-H09-136813, JP-A-2001-342255, a pamphlet of WO03/20828(A1), and JP-A-2009-185296. Examples of commercial products of a composition composed of a liquid oil material and such crosslinked organopolysiloxane polymer product having a hydrophilic group include, but are not particularly limited to, for example, KSG-210, KSG-240, and KSG-710, which are pastes mixed with silicone oil; KSG-310, KSG-320, KSG-330, KSG-340, KSG-820, KSG-830, KSG-840, and KSG-850Z which are pastes mixed with hydrocarbon oil or triglyceride oil (all of which are manufactured by Shin-Etsu Chemical Co., Ltd.).

In addition, the composition composed of a liquid oil material and the crosslinked organopolysiloxane having a hydrophilic group(s) may be blended in an amount of preferably 0.1 to 50% by mass, more preferably 1 to 30% by mass, per the whole amount of the cosmetic product.

The cosmetic product of the present invention may contain a silicone wax in accordance with an intended object. This silicone wax is preferably a polylactone-modified polysiloxane having bonded polylactone, which is a ring-opening polymerization product of a lactone compound having a ring of five or more atoms. Alternatively, this silicone wax is preferably an acrylic-modified polysiloxane with the molecule containing at least one functional group selected from a pyrrolidone group, a long-chain alkyl group, a polyoxyalkylene group, a fluoroalkyl group, and anionic groups, such as a carboxylic acid. Examples of commercial products of wax having a long-chain alkyl group include, for example, KP-561P and KP-562P (all of which are manufactured by Shin-Etsu Chemical Co., Ltd.).

The cosmetic product of the present invention may further contain, for example, a ingredient(s) used for ordinary cosmetic products, an oil-soluble gelation agent (organic-modified clay mineral), various powders, an antiperspirant, a moisturizer, an antimicrobial preservative, a fragrance, salts, antioxidant, a pH adjuster, a chelating agent, a cooling agent, an anti-inflammatory agent, a skincare component (such as whitening agent, cell activator, rough skin improver, blood circulation promoter, a skin astringent, an antiseborrheic agent), vitamins, amino acids, a nucleic acid, a hormone, an inclusion compound.

Examples of the oil-soluble gelation agent include one or more kinds of oil-soluble gelation agents selected from, for example, metal soaps, such as aluminum stearate, magnesium stearate, and zinc myristate; amino acid derivatives, such as N-lauroyl-L-glutamic acid and α,γ-di-n-butyl amine; dextrin fatty acid esters, such as dextrin palmitate ester, dextrin stearate ester, and dextrin 2-ethylhexanoate palmitate ester; sucrose fatty acid esters, such as sucrose palmitate ester and sucrose stearate ester; fructo-oligosaccharide fatty acid esters, such as fructo-oligosaccharide stearate ester and fructo-oligosaccharide 2-ethylhexanoate ester; benzylidene derivatives of sorbitol, such as monobenzylidene sorbitol and dibenzylidene sorbitol; organic-modified clay mineral, such as dimethyl benzyl dodecyl ammonium montmorillonite clay and dimethyl dioctadecyl ammonium hectorite.

Each of the various powders listed below indicates a surface-untreated powder of an organopolysiloxane represented by the average structural formula (1) of the present invention, and specific examples thereof include, for example, an inorganic powder, a color pigment, a pearl pigment, and a metal powder pigment.

Examples of the inorganic powder include titanium oxide, zirconium oxide, zinc oxide, cerium oxide, magnesium oxide, barium sulfate, calcium sulfate, magnesium sulfate, calcium carbonate, magnesium carbonate, talc, mica, kaolin, sericite, muscovite, synthetic mica, phlogopite, red mica, biotite, lithia mica, silicic acid, anhydrous silicic acid, aluminum silicate, magnesium silicate, aluminum magnesium silicate, calcium silicate, barium silicate, strontium silicate, metal tungstate, hydroxyapatite, vermiculite, Higilite, bentonite, montmorillonite, hectorite, zeolite, ceramic powder, dibasic calcium phosphate, alumina, aluminum hydroxide, boron nitride, silica, and silica silylate.

Examples of the color pigment include, for example, an inorganic red pigment such as iron oxide, iron hydroxide, and iron titanate; an inorganic brown pigment such as γ-iron oxide; an inorganic yellow pigment such as yellow iron oxide and loess; an inorganic black pigment such as black iron oxide and carbon black; an inorganic purple pigment such as manganese violet and cobalt violet; an inorganic green pigment such as chromium hydroxide, chromium oxide, cobalt oxide, and cobalt titanate; an inorganic blue pigment such as Prussian blue and ultramarine blue; a tar-based pigment that has been turned into a lake pigment; a natural pigment that has been turned into a lake pigment; and a synthetic resin powder pigment prepared by compounding these powders.

Examples of the pearl pigment include, for example, muscovite coated with titanium oxide, mica coated with titanium oxide, oxychloro bismuth, oxychloro bismuth coated with titanium oxide, talc coated with titanium oxide, fish scale foil, color mica coated with titanium oxide.

Examples of the metal powder pigment include, for example, aluminum powder, copper powder, and stainless powder.

The powders that can be added to the cosmetic product of the present invention preferably have a hydrophobic surface. The term hydrophobic refers to a state of not being dispersed in water. If the powder surface is hydrophobic from the beginning, the powder may be used as it is. As necessary, a powder that is hydrophobized in a manner as indicate below may be used. The use of such hydrophobic powder improves the uniformity of the cosmetic film, settlement upon application, and adhesiveness. In addition, the powders preferably have an average particle diameter of 200 µm or less. The particles constituting the powders are not particularly limited in regard to their forms or particle structures. The average particle diameter is preferably 200 µm or less, more preferably 100 µm or less, and even more preferably 50 µm or less. Here, the term "average particle diameter" as used herein refers to a volume-average particle diameter which can be measured using, for example, a laser scattering method.

The powders are preferably powders that are surface-hydrophobized using at least one treatment agent selected from silicone, fluorine compounds, silane coupling agents, titanium coupling agents, N-acylated amino acids, and metal soaps. Particularly, since the inorganic powder, color pigment, and pearl pigment have hydrophilic surfaces, it is preferred to use those that are subjected to surface hydrophobic treatment using at least one treatment agent selected from silicone, fluorine compounds, silane coupling agents, titanium coupling agents, N-acylated amino acids, and metal soaps.

Examples of the antiperspirant include, for example, aluminum chlorohydrate, aluminum chloride, aluminum sesquichlorohydrate, zirconyl hydroxychloride, aluminum zirconium hydroxychloride, aluminum zirconium glycine complex.

Examples of the moisturizer include sorbitol, xylitol, maltitol, polyethylene glycol, polysaccharides, hydroxyethyl cellulose, xanthan gum, hyaluronic acid, chondroitin sulfate, pyrrolidone carboxylate, polyoxyethylene methyl glucoside, polyoxypropylene methyl glucoside, egg-yolk lecithin, soybean lecithin, phosphatidylcholine, phosphatidylethanolamine, phosphatidylserine, phosphatidylglycerol, phosphatidylinositol, and sphingophospholipid.

Examples of the antimicrobial preservative include, for example, alkyl para-oxybenzoic acid esters (parabens), benzoic acid, sodium benzoate, sorbic acid, potassium sorbate, phenoxy ethanol. Examples of the antimicrobial include, for example, benzoic acid, salicylic acid, carbolic acid, sorbic acid, alkyl para-oxybenzoate ester, para-chloro-meta-cresol, hexachlorophene, benzalkonium chloride, chlorhexidine chloride, trichlorocarbanilide, photosensitizers, phenoxy ethanol.

The fragrance includes natural fragrance and synthetic fragrance. Examples of the natural fragrance include vegetable fragrances isolated from, for example, flower, leaf, material, and peel; and animal fragrances, such as musk (bisabolol) and civet. Examples of the synthetic fragrance include, for example, hydrocarbons, such as monoterpene; alcohols, such as aliphatic alcohols and aromatic alcohols; aldehydes, such as terpene aldehyde and aromatic aldehyde; ketones, such as alicyclic ketones; esters, such as terpene-based esters; lactones; phenols; oxides; nitrogen-containing compounds; and acetals.

Examples of the salt include, for example, inorganic salts, organic acid salts, amine salts, and amino acid salts. Examples of the inorganic salts include sodium salts, potassium salts, magnesium salts, calcium salts, aluminum salts, zirconium salts, barium salts, zinc salts, and the like of inorganic acid such as hydrochloric acid, sulfuric acid, carbonic acid, and nitric acid. Examples of the organic acid salts include salts of organic acids, such as acetic acid, dehydroacetic acid, citric acid, malic acid, succinic acid, ascorbic acid, and stearic acid. Examples of the amine salts and the amino acid salts include, for example, salts of amines such as triethanolamine; and salts of amino acids such as glutamic acid. Furthermore, it is also possible to use, for example, salts of hyaluronic acid, chondroitin sulfate; and aluminum zirconium glycine complex; as well as acid-alkali neutral salts, which are used in cosmetic preparations.

Examples of the antioxidant include, for example, tocopherol, p-t-butylphenol, butylhydroxyanisol, dibutylhydroxytoluene (BHT), and phytic acid.

Examples of the pH adjuster include, for example, lactic acid, citric acid, glycolic acid, succinic acid, tartaric acid, dl-malic acid, potassium carbonate, sodium bicarbonate, ammonium bicarbonate.

Examples of the chelating agent include, for example, alanine, sodium edetate, sodium polyphosphate, sodium metaphosphate, phosphoric acid.

Examples of the cooling agent include, for example, L-menthol and camphor.

Examples of the anti-inflammatory agent include, for example, allantoin, glycyrrhizinic acid and salt thereof, glycyrrhetinic acid and stearyl glycyrrhetinate, tranexamic acid, azulene.

Examples of the skincare component (such as a whitening agent, cell activator, rough skin improver, blood circulation promoter, skin astringent, antiseborrheic agent) include, for example, whitening agents, such as placenta extract, arbutin, glutathione, and a saxifragaceae extract; cell activators, such as royal jelly, photosensitive element, cholesterol derivative, and young calf blood extract; rough skin improvers; blood circulation promoters such as nonanoic acid vanillylamide, benzyl nicotinate, β-butoxyethyl nicotinate, capsaicin, zingerone, cantharides tincture, ichthammol, caffeine, tannic acid, α-borneol, nicotinic acid tocopherol, inositol hexanicotinate, cyclandelate, cinnarizine, tolazoline, acetylcholine, verapamil, cepharanthine, and γ-oryzanol; and skin astringents, such as zinc oxide and tannic acid; antiseborrheic agents such as sulfur and thianthol.

Examples of the vitamin include, for example, vitamin A derivatives such as vitamin A oil, retinol, retinol acetate, and retinol palmitate; vitamin B derivatives, including vitamin B₂, such as riboflavin, riboflavin butyrate, and flavin adenine nucleotide, vitamin B₆, such as pyridoxine hydrochloride salt, pyridoxine dioctanoate, and pyridoxine tripalmitate, vitamin B₁₂ and its derivative, and vitamin B₁₅ and its derivative; vitamin C derivatives, such as L-ascorbic acid, L-ascorbic acid dipalmitate ester, sodium L-ascorbic acid-2-sulfate, and dipotassium L-ascorbic acid phosphate diester; vitamin D derivatives, such as ergocalciferol and cholecalciferol; vitamin E derivatives, such as α-tocopherol, β-tocopherol, γ-tocopherol, dl-α-tocopherol acetate, dl-α-tocopherol nicotinate, and dl-α-tocopherol succinate; nicotinic acid derivatives, such as nicotinic acid, benzyl nicotinate, and nicotinic acid amide; vitamin H; vitamin P; pantothenic acid derivatives, such as calcium pantothenate, D-pantothenyl alcohol, pantothenyl ethyl ether, and acetyl pantothenyl ethyl ether; and biotin.

Examples of the amino acid include, for example, glycine, valine, leucine, isoleucine, serine, threonine, phenylalanine, arginine, lysine, aspartic acid, glutamic acid, cystine, cysteine, methionine, and tryptophan.

Examples of the nucleic acid include nucleic acids, such as deoxyribonucleic acid.

Examples of the hormone include, for example, estradiol, and ethenyl estradiol.

Examples of the inclusion compound include, for example, cyclodextrin.

The cosmetic product of the present invention includes cosmetic products in which the cosmetic product component(s) described above are blended, such as skincare cosmetic products, milky lotion, cream; make-up cosmetic products, including make-up foundation, concealer, white powder, liquid foundation, oil foundation, rouge, eye shadow, mascara, eyeliner, eye blow, and lipstick; hair cosmetic products, including shampoo, conditioner, treatment, and setting material; antiperspirant; UV protective cosmetic products, including sunscreen oil, sunscreen lotion, and sunscreen cream.

Moreover, as the form of these cosmetic products, various forms may be selected, such as liquid, milky lotion, cream, solid, paste, gel, powder, pressed, laminated, mousse, spray, and stick forms.

Further, as the type of these cosmetic products, various types may be selected, such as water-base, oil-base, water-in-oil emulsion, oil-in-water emulsion, non-aqueous emulsion, and multi-emulsion, including W/O/W and O/W/O.

### Examples

Hereinafter, the present invention will be specifically described with reference to Production examples, Examples of the inventive cosmetic, and Comparative Examples. However, the present invention is not limited to the following Examples. Here, unless otherwise expressly stated, "%" as described hereinunder refers to "% by mass", and % by mass of each component is expressed with reference to the total mass in each example being taken as 100%. The viscosity is a value at 25°C.

The polyoxyalkylene group-modified organopolysiloxanes as used in the production examples may be synthesized using a known method. Examples of such known method include those described in a pamphlet of WO2002/055588 (A1) and JP-A-2001-55307.

### (Production Working Example 1)

100 g of polyoxyalkylene-modified organopolysiloxane having a hydroxyl group at its end, represented by a structural formula (1'-1) as defined below, 50 g of toluene, and 5 g of triethylamine were put into a reactor and stirred, which was kept at a temperature of 80°C. 14 g of isocyanate having a hydrolyzable silyl group, represented by a structural formula (6-1) as defined below, was added dropwise to the mixture while keeping the temperature at or below 90°C, and the mixture was aged for 5 hours at 100°C. The reaction liquid was stripped under reduced pressure at 110°C to remove the solvent and other substances. It was confirmed from the ¹H-NMR spectrum that this reaction changed the chemical shifts of the N-(CH₂)₃-Si portion, originated from the isocyanate having a hydrolyzable silyl group, from 0.64 to 0.62, 1.70 to 1.58, and 3.26 to 3.14, respectively, and a colorless, transparent, liquid organopolysiloxane, represented by a structural formula (1-1) as defined below, was obtained. The water content of this organopolysiloxane was measured using a Karl Fischer moisture meter, and it turned out that the water content was 800 ppm. Furthermore, it did not turn into a gel after 30 days of storage at 25°C, demonstrating high stability.

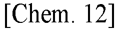

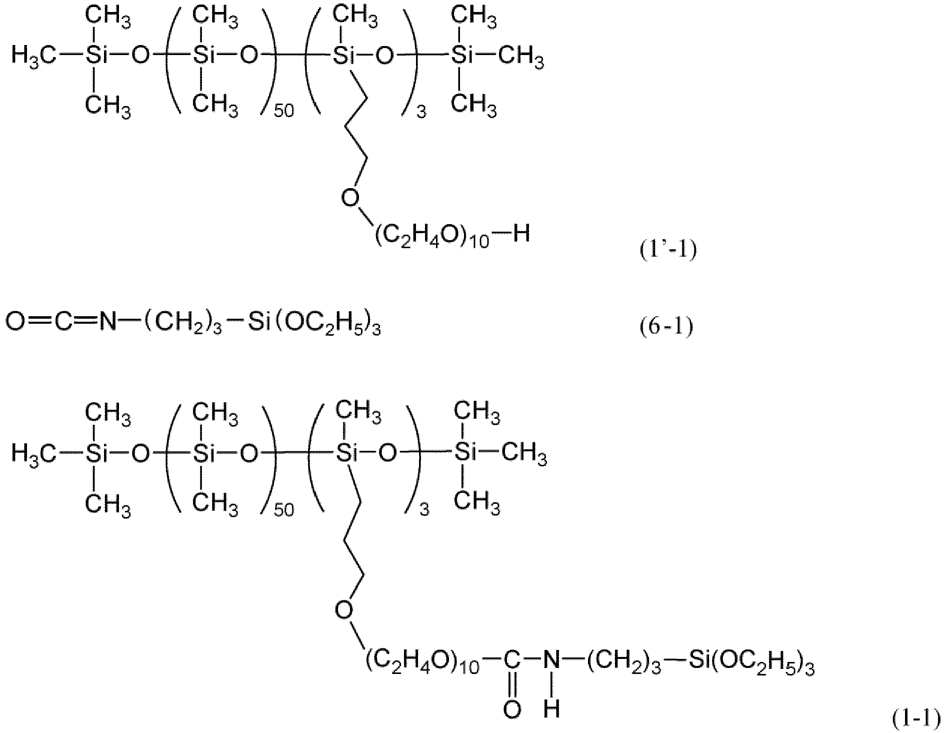

### (Production Working Example 2)

200 g of polyoxyalkylene-modified organopolysiloxane having a hydroxyl group at its end, represented by a structural formula (1'-2) as defined below, 50 g of toluene, and 5 g of triethylamine were put into a reactor and stirred, which was kept at a temperature of 80°C. 21 g of isocyanate having a hydrolyzable silyl group, represented by a structural formula (6-1) as defined below, was added dropwise to the mixture while keeping the temperature at or below 90°C, and the mixture was aged for 5 hours at 100°C. The reaction liquid was stripped under reduced pressure at 110°C to remove the solvent and other substances, and a colorless, transparent, liquid organopolysiloxane, represented by a structural formula (1-2) as defined below, was obtained. The water content of this organopolysiloxane was measured using a Karl Fischer moisture meter, and it turned out that the water content was 1,200 ppm. Furthermore, it did not turn into a gel after 30 days of storage at 25°C, demonstrating high stability.

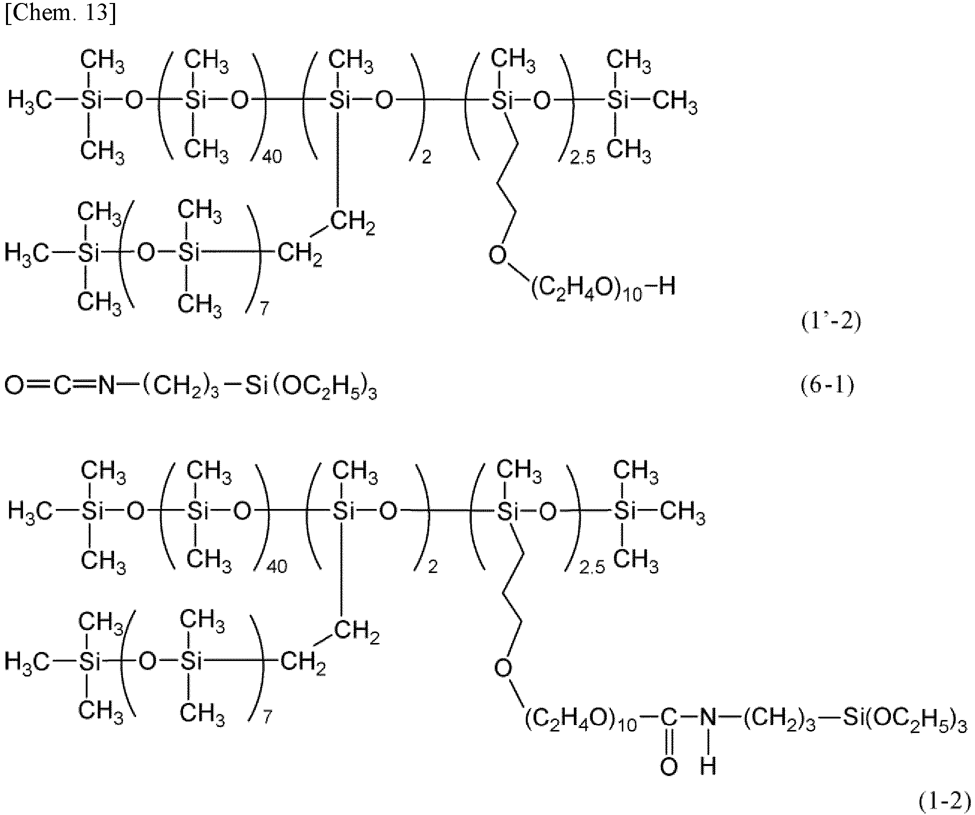

### (Production Working Example 3)

300 g of polyoxyalkylene-modified organopolysiloxane having a hydroxyl group at its **end, represented by a structural formula (1'-3)** as defined below, 100 g of toluene, and 10 g of triethylamine were put into a reactor and stirred, which was kept at a temperature of 80°C.

85 g of isocyanate having a hydrolyzable silyl group, represented by a structural formula (6-1) as defined below, was added dropwise to the mixture while keeping the temperature at or below 90°C, and the mixture was aged for 5 hours at 100°C. The reaction liquid was stripped under reduced pressure at 110°C to remove the solvent and other substances, and a colorless, transparent, liquid organopolysiloxane, represented by a structural formula (1-3) as defined below, was obtained. The water content of this organopolysiloxane was measured using a Karl Fischer moisture meter, and it turned out that the water content was 400 ppm. Furthermore, it did not turn into a gel after 30 days of storage at 25°C, demonstrating high stability.

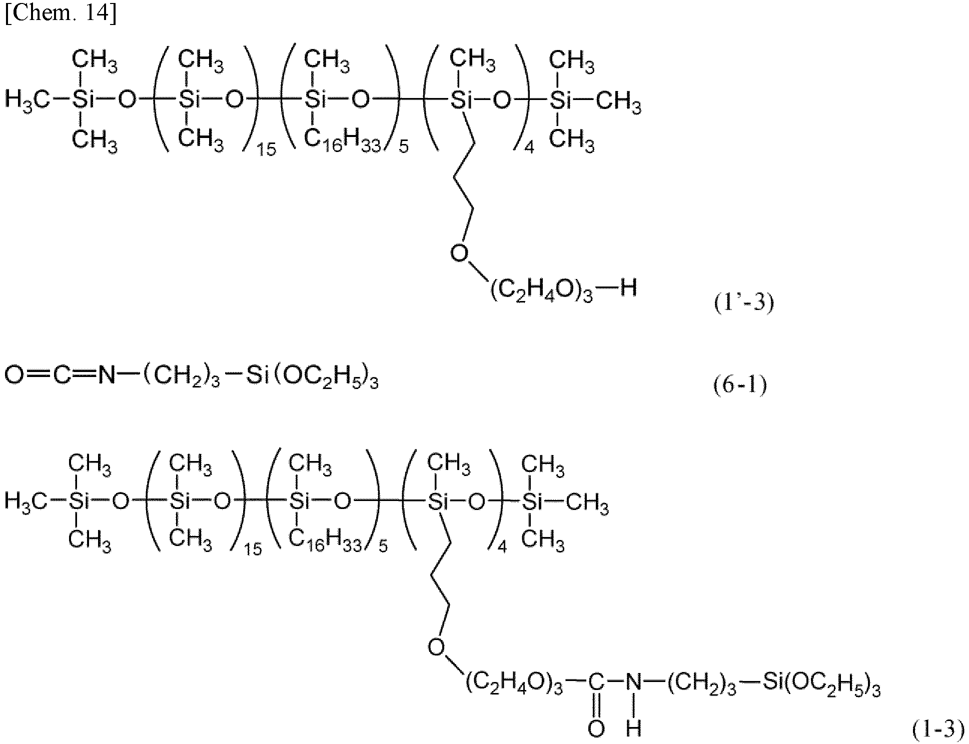

### (Production Working Example 4)

100 g of polyoxyalkylene-modified organopolysiloxane having a hydroxyl group at its end, represented by a structural formula (1'-4) as defined below, and 50 g of toluene were put into a reactor and stirred, which was kept at a temperature of 80°C. 24 g of isocyanate having a hydrolyzable silyl group, represented by a structural formula (6-4) as defined below, was added dropwise to the mixture while keeping the temperature at or below 90°C, and the mixture was aged for 5 hours at 100°C. The reaction liquid was stripped under reduced pressure at 110°C to remove the solvent and other substances, and a colorless, transparent, liquid organopolysiloxane, represented by a structural formula (1-4) as defined below, was obtained. The water content of this organopolysiloxane was measured using a Karl Fischer moisture meter, and it turned out that the water content was 1,800 ppm. Furthermore, it did not turn into a gel after 30 days of storage at 25°C, demonstrating high stability.

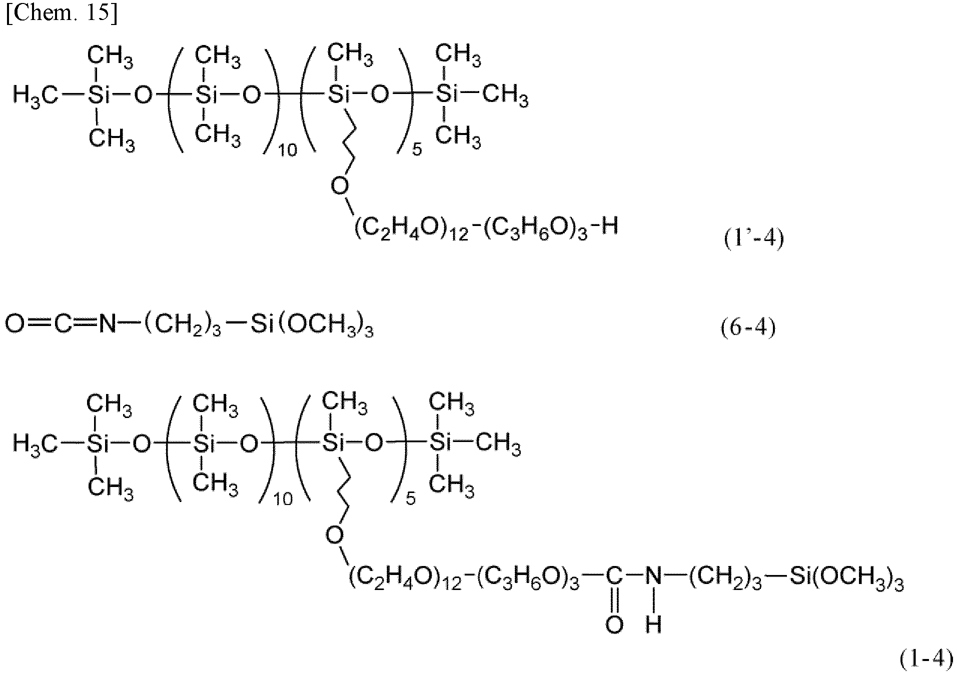

### (Production Working Example 5)

100 g of polyoxyalkylene-modified organopolysiloxane having a hydroxyl group at its end, represented by a structural formula (1'-5) as defined below, and 50 g of toluene were put into a reactor and stirred, which was kept at a temperature of 80°C. 6 g of isocyanate having a hydrolyzable silyl group, represented by a structural formula (6-4) as defined below, was added dropwise to the mixture while keeping the temperature at or below 90°C, and the mixture was aged for 5 hours at 100°C. The reaction liquid was stripped under reduced pressure at 110°C to remove the solvent and other substances, and a colorless, transparent, liquid organopolysiloxane, represented by a structural formula (1-5) as defined below, was obtained. The water content of this organopolysiloxane was measured using a Karl Fischer moisture meter, and it turned out that the water content was 1,000 ppm. Furthermore, it did not turn into a gel after 30 days of storage at 25°C, demonstrating high stability.

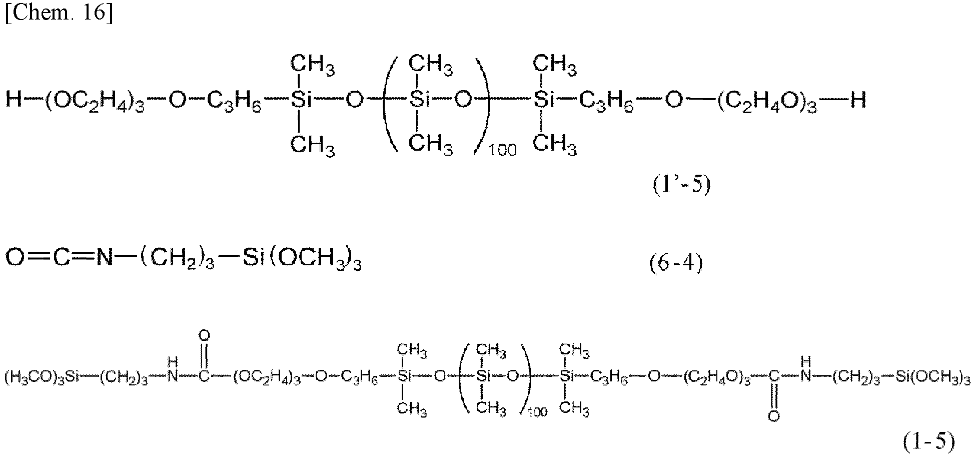

### (Production Working Example 6)

100 g of polyoxyalkylene-modified organopolysiloxane having a hydroxyl group at its end, represented by **a** structural formula (1'-6) as defined below, and 50 g of toluene were put into a reactor and stirred, which was kept at a temperature of 80°C. 5 g of isocyanate having a hydrolyzable silyl group, represented by a structural formula (6-4) as defined below, was added dropwise to the mixture while keeping the temperature at or below 90°C, and the mixture was aged for 5 hours at 100°C. The reaction liquid was stripped under reduced pressure at 110°C to remove the solvent and other substances, and a colorless, transparent, liquid organopolysiloxane, represented by a structural formula (1-6) as defined below, was obtained. The water content of this organopolysiloxane was measured using a Karl Fischer moisture meter, and it turned out that the water content was 1,000 ppm. Furthermore, it did not turn into a gel after 30 days of storage at 25°C, demonstrating high stability.

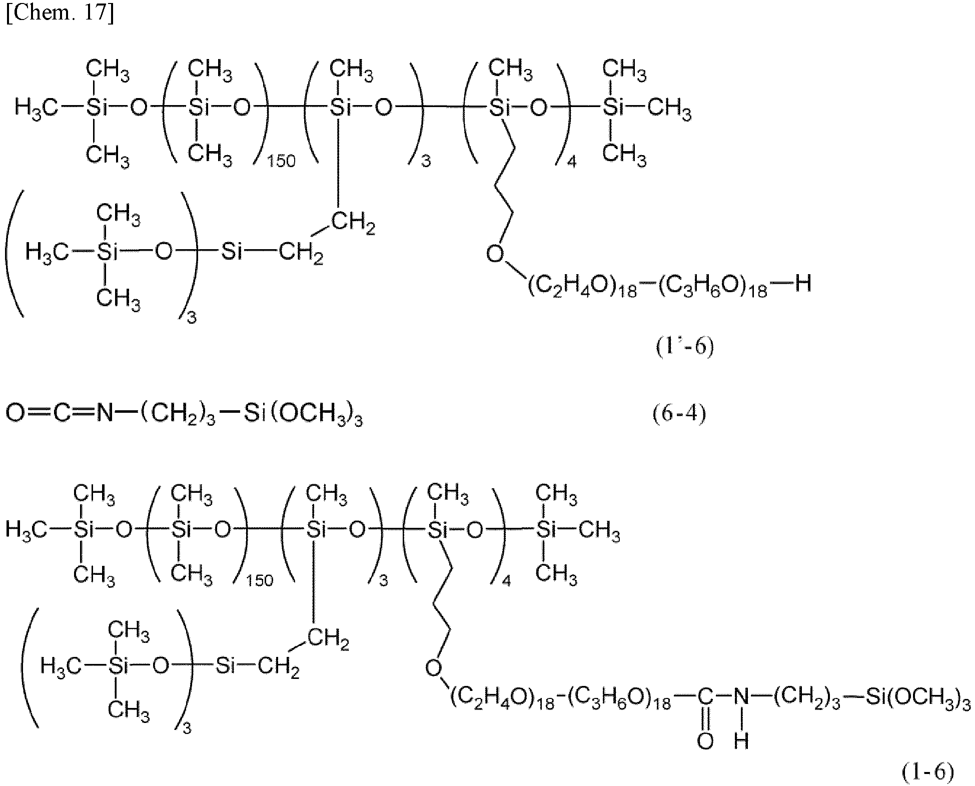

### (Production Comparative Example 1)

100 g of methylhydrogenorganopolysiloxane, represented by a structural formula (1"-1) as defined below, 50 g of 2-propanol, 0.03 g of a 3.0% ethanol solution of chloroplatinic acid, 37 g of allyl polyether, represented by a structural formula (1"-2) as defined below, and 13 g of vinyltriethoxysilane were put into a reactor and the mixture was aged at 85°C for 5 hours while stirring. The reaction liquid was stripped under reduced pressure at 110°C to remove the solvent and other substances, and a light yellow liquid organopolysiloxane, represented by a structural formula (1"-3) as defined below, was obtained. The water content of this organopolysiloxane was measured using a Karl Fischer moisture meter, and it turned out that the water content was 1,500 ppm. Furthermore, an increase in viscosity was observed after 30 days of storage at 25°C.

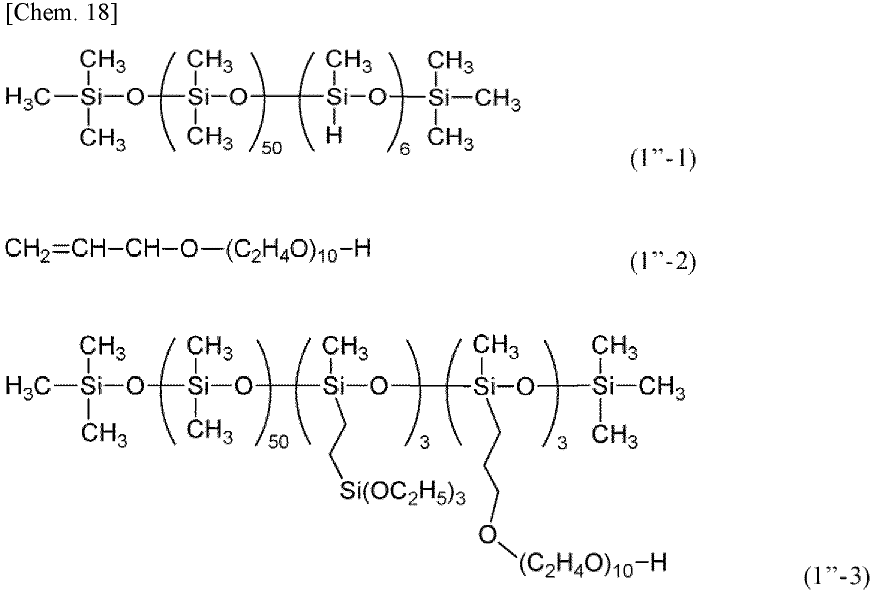

### (Production Comparative Example 2)

100 g of methylhydrogenorganopolysiloxane, represented by a structural formula (1"-4) as defined below, 50 g of 2-propanol, 0.03 g of a 3.0% ethanol solution of chloroplatinic acid, and 355 g of allyl polyether, represented by a structural formula (1"-5) as defined below, were put into a reactor and the mixture was aged at 85°C for 5 hours while stirring. The reaction liquid was stripped under reduced pressure at 110°C to remove the solvent and other substances, and an organopolysiloxane, represented by a structural formula (1"-6) as defined below, was obtained. The water content of this organopolysiloxane was measured using a Karl Fischer moisture meter, and it turned out that the water content was 400 ppm. Furthermore, gelation was observed after 1 day of storage at 25°C, demonstrating an unfavorable stability.

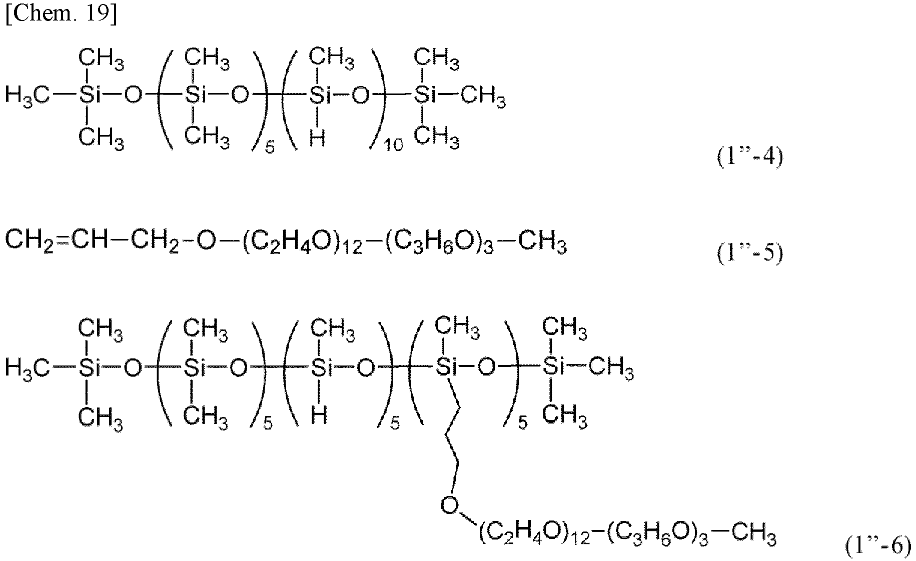

### (Production Examples 1 to 7), (Comparative Production Examples 1 and 2)

### Production Example 1

100 g of titanium oxide CR-50 (manufactured by Ishihara Sangyo Kaisha. LTD) was added to a mixer, and a solution of 5 g of the organopolysiloxane, obtained in Production Working Example 1, dissolved in 10 g of hexane was slowly added while stirring, and the mixture was stirred at 25°C for 1 hour. It was then heated at 100°C for 3 hours and dried to obtain a white powder. The results of ¹H-NMR showed no hint of unreacted ethoxy groups.

### Production Example 2

200 g of zinc oxide MZ-500 (manufactured by TAYCA Co., Ltd.) was added to a mixer, and a solution of 6 g of the organopolysiloxane, obtained in Production Working Example 2, dissolved in 10 g of hexane was slowly added while stirring, and the mixture was stirred at 25°C for 1 hour. It was then heated at 110°C for 3 hours and dried to obtain a white powder. The results of ¹H-NMR showed no hint of unreacted ethoxy groups.

### Production Example 3

500 g of black iron oxide BL-100P (manufactured by Titan Kogyo, Ltd) was added to a mixer, and a solution of 15 g of the organopolysiloxane, obtained in Production Working Example 3, dissolved in 15 g of ethanol was slowly added while stirring, and the mixture was stirred at 25°C for 3 hours. It was then heated at 90°C for 3 hours and dried to obtain a black powder. The results of ¹H-NMR showed no hint of unreacted ethoxy groups.

### Production Example 4

50 g of cellulose nanofiber was added to a mixer, and a solution of 6 g of the organopolysiloxane, obtained in Production Working Example 4, dissolved in 10 g of methanol was slowly added while stirring, and the mixture was stirred at 25°C for 3 hours. It was then heated at 100°C for 5 hours and dried to obtain a white powder. The results of ¹H-NMR showed no hint of unreacted methoxy groups.

### Production Example 5

100 g of spherical silica was added to a mixer, and a solution of 4 g of the organopolysiloxane, obtained in Production Working Example 1, dissolved in 10 g of hexane was slowly added while stirring, and the mixture was stirred at 25°C for 3 hours. It was then heated at 100°C for 5 hours and dried to obtain a white powder. The results of ¹H-NMR showed no hint of unreacted ethoxy groups.

### Production Example 6

100 g of spherical silica was added to a mixer, and a solution of 2 g of the organopolysiloxane, obtained in Production Working Example 5, dissolved in 10 g of hexane was slowly added while stirring, and the mixture was stirred at 25°C for 3 hours. It was then heated at 100°C for 5 hours and dried to obtain a white powder. The results of ¹H-NMR showed no hint of unreacted methoxy groups.

### Production Example 7

100 g of talc was added to a mixer, and a solution of 5 g of the organopolysiloxane, obtained in Production Working Example 6, dissolved in 10 g of hexane was slowly added while stirring, and the mixture was stirred at 25°C for 3 hours. It was then heated at 100°C for 5 hours and dried to obtain a white powder. The results of ¹H-NMR showed no hint of unreacted methoxy groups.

### Comparative Production Example 1

100 g of titanium oxide CR-50 (manufactured by Ishihara Sangyo Kaisha. LTD) was added to a mixer, and a solution of 5 g of the organopolysiloxane, obtained in Comparative Production Example 1, dissolved in 10 g of hexane was slowly added while stirring, and the mixture was stirred at 25°C for 1 hour. It was then heated at 100°C for 3 hours and dried to obtain a white powder. The results of ¹H-NMR showed the presence of residual unreacted ethoxy groups.

### Comparative Production Example 2

50 g of cellulose nanofiber was added to a mixer, and a solution of 6 g of the organopolysiloxane, obtained in Comparative Production Example 2, dissolved in 10 g of methanol was slowly added while stirring, and the mixture was stirred at 25°C for 3 hours. It was then heated at 100°C for 5 hours and dried to obtain a white powder. The results of ¹H-NMR showed the presence of residual unreacted hydrosilyl groups.

### (Working Examples 1 to 4 and Comparative Examples 1 to 5)

Emulsified creams obtained with the formulations shown in the table below were each produced using a conventional method to evaluate their stability over time and usability.

### [Method for evaluating stability over time]

For stability over time, the emulsion creams obtained with the formulations shown in the table below were each left to stand at 25°C for one month, and then the presence or absence of emulsion separation was visually confirmed. Those showing separation were marked with ×, while those showing no hint of separation were marked with ⊚.

### [Sensory evaluation method]

To evaluate usability, approximately 5 g of the emulsion cream obtained with the formulation shown in Table 1 below was applied to the skin of each five panelists to perform sensory evaluation on the spreadability and non-stickiness upon application. The term "spreadability upon application" as used herein refers to the extent or area that was physically stretched. In addition, stickiness was evaluated based on the presence or absence of adhesion. The results are shown according to the following evaluation criteria, depending on the number of panelists who answered the result as "effective."

### [Evaluation Criteria]

⊚: 4 to 5 people answered the result as effective
∘: 3 people answered the result as effective
△: 2 people answered the result as effective
×: Only one or no people answered the result as effective

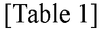

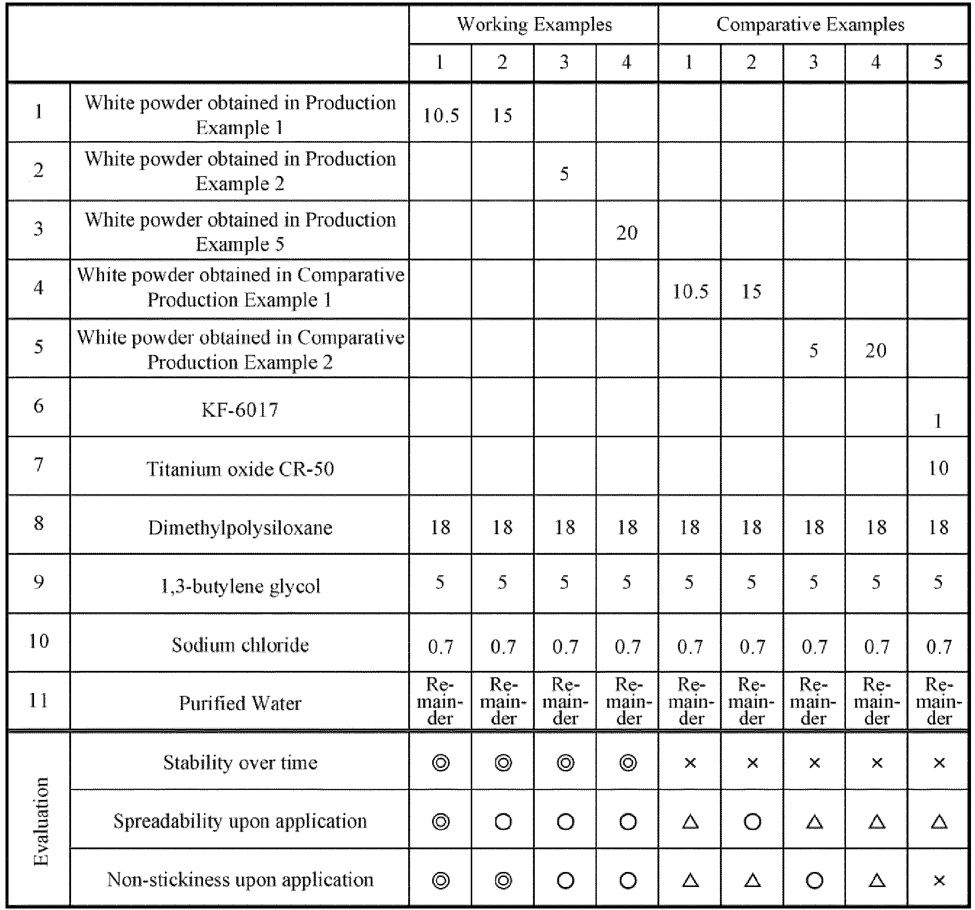

The formulations of the ingredients shown in Table 1 were adjusted to have a total amount of 100.
KF-6017: Polyoxyethylene-methylpolysiloxane copolymer, manufactured by Shin-Etsu Chemical Co., Ltd.
CR-50: Titanium oxide, manufactured by Ishihara Sangyo Kaisha, Ltd.
Dimethylpolysiloxane: Dynamic viscosity of 6 mm²/s at 25°C

As shown in Table 1, it was indicated that the emulsified creams of Working Examples 1 to 4 according to the present invention were products in which the treated powders obtained in the production examples work as emulsifiers and form a stable water-in-oil Pickering emulsion, and the emulsified creams demonstrated excellent stability over time, exhibited favorable spreadability upon application, and showed no hint of stickiness.

Meanwhile, those of the comparative examples 1 to 5 demonstrated poor stability over time and exhibited inferior usability upon application. As indicated from the above, it was found that cosmetic products using the treated powder of the present invention have excellent stability over time, and exhibit excellent usability as well.

### [Working Example 5]

### O/W Sunscreen

### <Preparation of cosmetic product>

A: Ingredients (9) to (16) were uniformly mixed at 85°C.
B: Ingredients (1) to (6) were uniformly mixed at 85°C.
C: At 80°C, the resultant product of step B above and ingredients (7) and (8) were added to the resultant product of step A above, emulsified, and cooled slowly to obtain an O/W sunscreen.

| Ingredients | Mass (%) |
|---|---|
| (1) Polyglyceryl-10 Laurate | 1.5 |
| (2) Glyceryl Stearate SE | 3 |
| (3) Behenyl alcohol | 3 |
| (4) Ethylhexyl methoxycinnamate | 5 |
| (5) Isononyl isononanoate | 5 |
| (6) Coco-Caprylate/Caprate | 5 |
| (7) Dispersion of silicone-treated zinc oxide fine particle (Note 1) | 10 |
| (8) Dispersion of metal soap-treated titanium oxide fine particle (Note 2) | 10 |
| (9) Hydroxyethyl cellulose | 0.2 |
| (10) 1,3-butylene glycol | 10 |
| (11) Ethanol | 6 |
| (12) Ethylhexylglycerin | 0.2 |
| (13) Bisabolol | 0.2 |
| (14) White powder obtained in Production Example 4 | 10 |
| (15) White powder obtained in Production Example 6 | 2 |
| (16) Purified water | Remainer |
| Total amount | 100.0 |

| | |
|---|---|
| (Note 1) SPD-Z5 manufactured by Shin-Etsu Chemical Co., Ltd. (Note 2) SPD-T5 manufactured by Shin-Etsu Chemical Co., Ltd. | |

The resultant O/W sunscreen was confirmed to have enhanced stability over time, no stickiness, and excellent usability.

### [Working Example 6]

### Powder foundation

### <Preparation of cosmetic product>

A: Ingredients 1 to 3 were uniformly mixed
B: Ingredients 4 to 12 were uniformly mixed
C: The product obtained in step A above was added to the product obtained in step B above, which was uniformly mixed using a Henschel mixer. The resulting powder was passed through a 100 mesh screen, and then pressed into a metal dish using a mold to obtain a powder foundation.

| Ingredients | | Mass |
|---|---|---|
| (%) | | |
| 1. | 2-Ethylhexyl p-methoxycinnamate | 4 |
| 2. | Diphenylsiloxy phenyl trimethicone (Note 1) | 4.5 |
| 3. | Glyceryl triethylhexanoate | 1.5 |
| 4. | Barium sulfate | 10 |
| 5. | White powder obtained in Production Example 5 | 5 |
| 6. | Cross-linked dimethylpolysiloxane (Note 2) | 4 |
| 7. | Mica treated with alkylsilicone-branched silicone (Note 3) | 30 |
| 8. | Talc treated with alkylsilicone-branched silicone (Note 3) | 33.3 |
| 9. | Titanium oxide treated with alkylsilicone-branched silicone (Note 4) | 6 |
| 10. | Yellow iron oxide treated with alkylsilicone-branched silicone (Note 4) | 1.0 |
| 11. | Red iron oxide treated with alkylsilicone-branched silicone (Note 4) | 0.5 |
| 12. | Black powder obtained in Production Example 3 | 0.2 |
| Total amount | | 100.0 |

| | | |
|---|---|---|
| (Note 1): KF-56A manufactured by Shin-Etsu Chemical Co., Ltd. (Note 2): KSG-15 manufactured by Shin-Etsu Chemical Co., Ltd. (Note 3): Treatment with KF-9909 manufactured by Shin-Etsu Chemical Co., Ltd. (Note 4): KTP-09-Series manufactured by Shin-Etsu Chemical Co., Ltd. | | |

The obtained powder foundation was a foundation having favorable spreadability and excellent usability.

### [Working Example 7]

### Milky lotion

### <Preparation of cosmetic product>

Ingredients (1) to (9) were uniformly mixed.

| Ingredients | Mass (%) |
|---|---|
| (1) Ethanol | 5.00 |
| (2) Glycerin | 4.00 |
| (3) 1,3-butylene glycol | 3.00 |
| (4) Purified water | Remainder |
| (5) Carboxy vinyl polymer (1% aqueous solution) | 15.00 |
| (6) Sodium hydroxide | 0.05 |
| (7) Xanthan gum (2% aqueous solution) | 10.00 |
| (8) Edetate disodium | 0.05 |
| (9) White powder obtained in Production Example 4 | 10.00 |
| Total Amount | 100.00 |

The obtained milky lotion had enhanced stability over time, light spreadability, and stretchability, and exhibited excellent usability.

### [Working Example 8]

### Mascara

### <Preparation of cosmetic product>

A: Ingredients 1 to 7 were mixed under heat.
B: Ingredients 8 to 11 were treated with a three-roll mill.
C: Ingredients 12 to 14 were added to the product obtained in step B above and heated.
D: The product obtained in step C above was added to the product obtained in step A above and emulsified, which was then cooled to obtain a mascara.

| Ingredients | Mass (%) |
|---|---|
| 1. Stearic acid | 2.0 |
| 2. Carnauba wax | 2.0 |
| 3. White beeswax | 7.0 |
| 4. Polyoxyethylene Sorbitan Monooleate (20E.O.) | 0.2 |
| 5. Sorbitan Sesquioleate | 0.4 |
| 6. Triethanolamine | 0.5 |
| 7. Sucrose fatty acid ester | 1.5 |
| 8. White powder obtained in Production Example 1 | 2.0 |
| 9. Black powder obtained in Production Example 3 | 6.0 |
| 10. White powder obtained in Production Example 7 | 3.0 |
| 11. Propylene glycol | 7.0 |
| 12. Preservative | Optimum amount |
| 13. Polyethyl acrylate ester emulsion | 50.0 |
| 14. Purified water | Remainder |
| Total amount | 100.0 |

The obtained mascara was a mascara that had enhanced stability over time and favorable spreadability, and exhibited excellent coating properties.

## Claims

1. An organopolysiloxane represented by the following average structural formula (1): 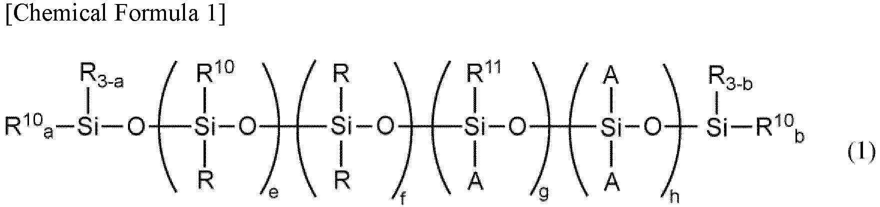
wherein, in the formula (1), each R independently represents a group selected from a hydrogen atom, a hydroxy group, an alkyl group having 1 to 30 carbon atoms, a fluoroalkyl group having 1 to 12 carbon atoms, an aryl group having 6 to 12 carbon atoms, and an aralkyl group having 7 to 12 carbon atoms, and each R¹⁰ independently represents a group defined by the following average structural formula (2): 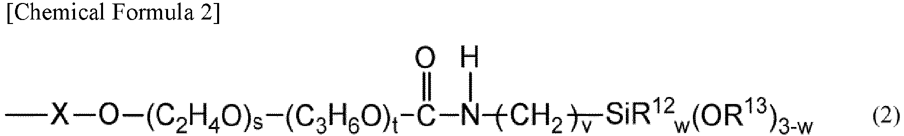 wherein, in the formula (2), X is a divalent hydrocarbon group having 1 to 10 carbon atoms, s is a number of 1 to 100, t is a number of 0 to 50, the bonding arrangement of oxyalkylene units each identified by s and t in brackets may be blocked or random, v is a number of 1 to 10, each R¹² independently represents a group selected from an alkyl group having 1 to 6 carbon atoms and an aryl group having 6 to 12 carbon atoms, each R¹³ independently represents a hydrogen atom or a monovalent hydrocarbon group having 1 to 6 carbon atoms, and w is a number of 0 to 2;
wherein R¹¹ is a group selected from R¹⁰ or R, and each A independently represents an organopolysiloxane segment defined by the following average structural formula (3) or (4): 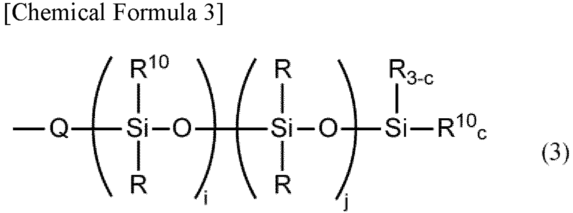 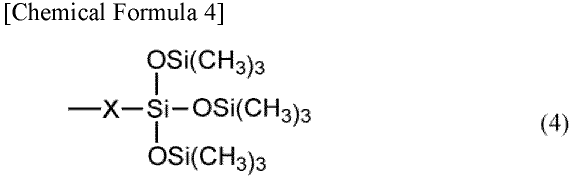 wherein, in the formula (3), R and R¹⁰ are as defined above, c is a number of 0 to 3, i is a number of 0 to 10, j is a number of 0 to 100, and Q is an oxygen atom or a divalent hydrocarbon group having 1 to 3 carbon atoms, and in the formula (4), X is as defined above, and the bonding arrangement of siloxane units each identified by i and j in brackets may be blocked or random, and
wherein, in the formula (1), each of a and b independently represents a number of 0 to 3, e is a number of 0 to 50, f is a number of 0 to 2,000, g is a number of 0 to 10, h is 0 or 1, the bonding arrangement of siloxane units each identified by e, f, g and h in brackets may be blocked or random, and one or more R¹⁰s are present in the formula (1).

2. A powder treatment agent comprising the organopolysiloxane according to claim 1, wherein the agent contains water in an amount of 2,000 ppm or less.

3. A powder surface treated with the powder treatment agent according to claim 2.

4. The treated powder according to claim 3, wherein the powder is an organic or inorganic powder.

5. A cosmetic product containing the treated powder according to claim 4.

6. The cosmetic product according to claim 5, further comprising water and an oil agent.
